# EUROPEAN PATENT APPLICATION

(11) **EP 4 385 533 A1**
(43) Date of publication of application: **19.06.2024**
(21) Application number: 22857568.4
(22) Date of filing: 29.07.2022
(51) Int. Cl.: A61L 27/52, A61L 27/58, A61L 27/50, A61L 27/26, A61L 27/20

(54) **BIOLOGICAL POLYSACCHARIDE HYDROGEL, PREPARATION METHOD THEREFOR AND APPLICATION THEREOF**

(30) Priority: 14.08.2021 CN 202110933233
(71) Applicant: QINGDAO HEALTH OCEAN BIOPHARMACEUTICAL CO., LTD., Laoshan District Qingdao, Shandong 266104 (CN)
(72) Inventor: HAN, Baoqin, Qingdao, Shandong 266003 (CN); WANG, Shuo, Qingdao, Shandong 266003 (CN); LIU, Wanshun, Qingdao, Shandong 266003 (CN)
(74) Representative: Vossius & Partner Patentanwälte Rechtsanwälte mbB
(86) International application number: PCT/CN2022/108927
(87) International publication number: WO 2023/020256

(57) **Abstract**

The present invention relates to a biological polysaccharide hydrogel, a preparation method therefor and an application thereof. The biological polysaccharide hydrogel of the present invention is formed by means of the mixing and reaction of two solutions; the first solution is a polysaccharide derivative solution having an aldehyde on a side chain; the second solution is a polysaccharide derivative solution having a primary amine on a main chain or a side chain, or a small molecule compound solution having two or more primary amines. The preparation method of the present invention comprises preparing the first solution and the second solution, and then mixing the first solution and the second solution to form a transparent biological polysaccharide hydrogel. The biological polysaccharide hydrogel of the present invention can be applied as a vitreous substitute.

## Description

### FIELD OF THE INVENTION

The present invention relates to an ophthalmic biomedical material, in particular to a biological polysaccharide hydrogel, a preparation method thereof and an application thereof as a vitreous substitute.

### BACKGROUND OF THE INVENTION

The vitreous body inside the eyeball is a colorless, transparent colloid that fills the space between the lens and the retina. It occupies more than 2/3 of the volume of the eyeball and is mainly composed of water, collagen protein, hyaluronic acid, and proteoglycans. Water constitutes about 99% of the volume of the vitreous body. Hyaluronic acid and collagen protein interact with each other and bind with water to maintain the stability of the vitreous body. The vitreous body has high transparency, allowing more than 90% of visible light to pass through. It plays an important role in maintaining the shape of the eyeball, regulating intraocular oxygen tension, providing shock absorption, and maintaining the position of the retina and lens. Vitrectomy is a common surgery used to treat many eye disorders such as retinal diseases, retinal detachment, macular hole, etc. After vitrectomy, the vitreous cavity needs to be filled with a vitreous substitute, which presses against the retina to maintain its correct position and maintain the shape of the eyeball. Therefore, the vitreous substitute is an important factor affecting the effectiveness of the surgery.

Currently, the vitreous substitutes used in clinical practice mainly include gas, silicone oil, and perfluorocarbon liquid. These substitutes are used for vitreous filling in different surgical situations but still have various complications. For example, the absorption of gas vitreous filler is relatively fast, the effective pressure-holding time is short, and patients need to maintain a certain position after surgery. Perfluorocarbon liquid also absorbs quickly, with an effective pressure-holding time of about 4 weeks. Commonly used silicone oil filler has a long pressure-holding time, but it is not absorbed by body tissues. There are many complications caused by silicone oil emulsification, such as glaucoma, cataracts, etc., so silicone oil needs to be removed by a second operation. At present, there is no ideal vitreous substitute.

As a vitreous substitute, high light transmissivity, good viscoelasticity, effective pressure-holding time, and non-toxic side effects on eye tissues are extremely important. The patent application with the publication number CN 102762647 A discloses a composition including a hydrogel polymer, which contains oxidized hyaluronic acid and diimide, where diimide cross-links with oxidized hyaluronic acid, forming a transparent and colorless substance that transitions from liquid to gel, serving as an eyeball vitreous substitute. The patent application with the publication number CN 110652610 A discloses a complete set of raw materials for a high-cohesive double-crosslinked artificial vitreous body. Its first component is aldehyde-modified hyaluronic acid, which is oxidized ring-opened hyaluronic acid, and the second component is aminated hyaluronic acid. The first and second agents are mixed and crosslinked to prepare a high-cohesive double-crosslinked artificial vitreous body. The shared feature of the aforementioned two patent applications is using ring-opened oxidized hyaluronic acid as an aldehyde donor to form a gel by crosslinking with dihydrazide or diamine.

The patent application with the publication number CN 105833344 A discloses the use of an injectable hydrogel in the preparation of intraocular fillers. The injectable hydrogel consists of two agents. The first agent is a gel solution containing oxidized polysaccharides, and the second agent is a gel solution containing chitosan derivatives and/or collagen. A cross-linking reaction occurs between the dialdehyde group of the oxidized polysaccharides and the amino group of the chitosan derivatives and/or collagen, forming a viscoelastic hydrogel.

All of the aforementioned existing technologies use ring-opened oxidized hyaluronic acid to form a gel. However, ring-opening oxidation significantly reduces the molecular weight of hyaluronic acid, leading to its rapid degradation in the body and thus shortening the effective pressure-holding time when used as a vitreous substitute. As a vitreous substitute, it is extremely important to prolong the degradation time in the body and extend its effective pressure-holding time on the retina to ensure that the retina is repositioned and prevent retinal detachment.

### SUMMARY OF THE INVENTION

In view of the above problems, the first object of this invention is to provide a biological polysaccharide hydrogel suitable for use as a vitreous substitute, which has high light transmissivity, good viscoelasticity, extended degradation time, effectively improved pressure-holding time, and non-toxic side effects on eye tissues, in order to make up for the deficiencies in the existing technologies.

Another object of this invention is to provide a method for preparing a biological polysaccharide hydrogel, as well as the use of the biological polysaccharide hydrogel in the preparation of a vitreous substitute.

The technical solution of this invention, while considering the excellent light transmissivity, viscoelasticity, and tissue non-toxicity of the hydrogel as a vitreous substitute, focuses on solving the problem of extending the degradation time of the hydrogel to lengthen the pressure-holding time on the retina.

The biological polysaccharide hydrogel of this invention consists of two agents. The first agent is a polysaccharide derivative solution with an aldehyde group on a side chain, and the second agent is a polysaccharide derivative solution with a primary amino group on a main chain or on a side chain, or a small molecule compound solution with two or more primary amino groups.

The first agent, a polysaccharide derivative with an aldehyde group on a side chain, is formed from a polysaccharide derivative with an o-dihydroxyl group on a side chain through oxidation. Under controlled reaction conditions, the oxidizing agent preferentially oxidizes the o-dihydroxyl groups on the side chains of the polysaccharide to form aldehyde groups, but does not oxidize the main chain of the polysaccharide, thereby ensuring that the polysaccharide molecules are not ring-opened or degraded, and the integrity of the main chain of the polysaccharide is maintained. At the same time, polysaccharide derivatives with grafted structures on the side chains reduce the specific recognition of polysaccharides by bio-enzymes, thereby enhancing the resistance of polysaccharide derivatives to biodegradation.

The second agent, a polysaccharide derivative with a primary amino group on a main chain or on a side chain, includes a polysaccharide derivative with a primary amino group on a main chain and a polysaccharide derivative with a primary amino group on a side chain. The polysaccharide derivative with a primary amino group on a main chain is characterized by having a primary amino group on a main chain of the polysaccharide derivative, together with a grafted structure on a side chain. The polysaccharide derivative with a primary amino group on a side chain is characterized by having a primary amino group on a side chain of the polysaccharide derivative. That is, both the polysaccharide derivative with a primary amino group on a main chain and the polysaccharide derivative with a primary amino group on a side chain have side-chain grafted structures. These side-chain grafted structures reduce the specific recognition of polysaccharides by bio-enzymes, thereby enhancing the resistance of polysaccharide derivatives to biodegradation.

When the first agent, a solution of a polysaccharide derivative with an aldehyde group on a side chain, is mixed with the second agent, a solution of a polysaccharide derivative with a primary amino group on a main chain or a side chain, a cross-linking reaction occurs and forms a hydrogel. On one hand, because the main chain of the polysaccharide derivative acting as the aldehyde donor is not oxidized and ring-opened, there is no oxidative degradation of the molecular chain. On the other hand, because the polysaccharide derivatives acting as aldehyde donor and amino donor both have side-chain grafted structures, the presence of these side-chain grafted structures reduces the specific recognition of polysaccharides by bio-enzymes, thereby enhancing the resistance of polysaccharide derivatives to biodegradation. When the first agent, a solution of a polysaccharide derivative with an aldehyde group on a side chain, is mixed and crosslinked with the second agent, a solution of a small molecule compound with two or more primary amino groups, to form a hydrogel, the main chain of the polysaccharide derivative acting as the aldehyde donor is not oxidized and ring-opened, preventing oxidative degradation of the molecular chain. At the same time, the polysaccharide derivative acting as the aldehyde donor has side-chain grafted structures. When the polysaccharide derivative acting as the aldehyde donor crosslinks with the small molecule compound with primary amino groups, the crosslinked structure of the small molecule compound further reduces the specific recognition of polysaccharides by bio-enzymes, thereby enhancing the resistance of polysaccharide derivatives to biodegradation. By enhancing the biodegradation resistance of the crosslinked polysaccharide hydrogel, the effective pressure-holding time of the hydrogel on the retina is extended. Therefore, the purpose of providing suitable hydrogel for use as a vitreous substitute is achieved.

The specific technical solution of the invention is described below.

In one aspect, the invention relates to a biological polysaccharide hydrogel, which is formed by the reaction of a first solution and a second solution, wherein the first solution is a solution of a polysaccharide derivative with an aldehyde group on a side chain, and the second solution is a solution of a polysaccharide derivative with a primary amino group on a main chain or on a side chain, or a solution of a small molecule compound with two or more primary amino groups.

The first solution comprises 0.5% to 15% by weight of a polysaccharide derivative with an aldehyde group on a side chain, and the second solution comprises 0.5% to 15% by weight of a polysaccharide derivative with a primary amine group on a main chain or on a side chain, or 0.2% to 30% by weight of a small molecule compound with two or more primary amine groups; and the first and second solutions comprise a solvent selected from the group consisting of sterile water, physiological saline, physiological balanced solution, and glucose solution.

The polysaccharide derivative with an aldehyde group on a side chain in the first solution is an aminopolysaccharide derivative with an aldehyde group on a side chain or is a carboxypolysaccharide derivative with an aldehyde group on a side chain. the aminopolysaccharide derivative with an aldehyde group on a side chain is an aminopolysaccharide derivative whose amino group is modified to form a side chain having an aldehyde group, and the carboxypolysaccharide derivative with an aldehyde group on a side chain is a carboxypolysaccharide derivative whose carboxyl group is modified to form a side chain having an aldehyde group. The aminopolysaccharide derivative with an aldehyde group on a side chain or the carboxypolysaccharide derivative with an aldehyde group on a side chain is formed by oxidation of an aminopolysaccharide whose amino group has been modified to form a side chain having an o-dihydroxy group, or a carboxypolysaccharide whose carboxyl group has been modified to form a side chain having an o-dihydroxy group.

The amino polysaccharide derivative in the first solution of this invention includes hydroxyethyl chitosan, hydroxypropyl chitosan, hydroxybutyl chitosan, carboxymethyl chitosan, or carboxyethyl chitosan; and the carboxypolysaccharide derivative includes a chitosan derivative with a carboxyl group, a chitin derivative with a carboxyl group, a hyaluronic acid with a carboxyl group or a hyaluronic acid derivative with a carboxyl group, chondroitin sulfate, heparin, or sodium alginate.

The small molecule compound with two or more primary amino groups according to the invention is an aliphatic or aromatic small molecule compound with two or more primary amine groups, including ethylenediamine, propylenediamine, butylenediamine, aminobutanamide, glutamine, alanyl-glutamine, lysine or other basic amino acids, small molecule compounds with a dihydrazide structure, diaminobenzoic acid or diaminophenylacetic acid.

The small molecule compound with a dihydrazide structure according to the invention includes carbohydrazide, oxalyl dihydrazide, propanedioyl dihydrazide, butanedioyl dihydrazide, adipic dihydrazide or sebacic dihydrazide.

The polysaccharide derivative with a primary amino group on a main chain in the second solution of this invention is an aminopolysaccharide derivative that has a primary amino group on a main chain and also has a grafted structure on a side chain. The polysaccharide derivative with a primary amino group on a side chain is a polysaccharide derivative that has a carboxyl group on a main chain and has a primary amino group on a side chain. The polysaccharide derivative with a primary amino group on a side chain is formed by condensation of a carboxyl group of a carboxypolysaccharide with one amino group of a small molecule compound having two or more primary amino groups.

The preparation method of the above-mentioned biological polysaccharide hydrogel includes the following steps:
(1) providing a first solution, which is a sterilized solution comprising 0.5% to 15% by weight of a polysaccharide derivative with an aldehyde group on a side chain, and adjusting the pH of the first solution to 3.5 to 7, wherein the polysaccharide derivative with an aldehyde group on a side chain is formed by the reaction of an aqueous solution of a polysaccharide derivative having a grafted o-dihydroxyl group with an oxidizing agent;
(2) providing a second solution selected from the group consisting of:
   i) a sterilized solution comprising 0.2% to 30% by weight of a small molecule compound with two or more primary amino groups;
   ii) a sterilized solution comprising 0.5% to 15% by weight of a polysaccharide derivative with a primary amino group on a main chain;
   iii) a sterilized solution comprising 0.5% to 15% by weight of a polysaccharide derivative with a primary amino group on a side chain;
   and adjusting the pH of the second solution to 7 to 9, and measuring the mole percentage content of primary amino groups therein;
(3) mixing the first solution with the second solution: adding the first solution obtained in step (1) and the second solution obtained in step (2) respectively into tube A and tube B of a sterilized dual syringe, pushing them out from the syringe for mixing and forming a transparent biological polysaccharide hydrogel by mixing the first solution and the second solution.

The polysaccharide derivative with an aldehyde group on a side chain in step (1) is prepared by the following method: providing an aqueous solution comprising 0.5% to 15% by weight of a polysaccharide derivative having a grafted o-dihydroxyl group, adding the oxidizing agent, wherein the molar ratio of the o-dihydroxyl group to the oxidizing agent is 1: (0.3 to 3), stirring the reaction mixture at room temperature or lower temperature and under dark condition for 2 minutes to 5 hours, adding an ethylene glycol solution to terminate the reaction for 0.5 to 2 hours, and performing dialysis of the reaction product and freeze drying, or precipitating the reaction product with ethanol, washing, precipitating, and drying.

The polysaccharide derivative having a grafted o-dihydroxyl group in step (1) is prepared by the following method: providing an aqueous solution comprising 0.5% to 15% by weight of an aminopolysaccharide and an aqueous solution comprising 0.2% to 30% by weight of a small molecule compound with a carboxyl group and an o-dihydroxyl group respectively, adding the carboxyl activating agents 1-(3-dimethylaminopropyl)-3-ethylcarbodiimide hydrochloride and N-hydroxysuccinimide to the small molecule compound solution, and adjusting the pH to 3.5 to 7, stirring the resulting solution to react for 0.5 to 3 hours; adding the obtained small molecule compound solution to the aminopolysaccharide solution to form a reaction system, stirring and adjusting the pH of the reaction system to 7 to 9, and stirring the reaction system to react for 24 hours.

The small molecule compound with a carboxyl and an o-dihydroxyl group in step (1) includes 2,3-dihydroxypropanoic acid, 2,3,4-trihydroxybutanoic acid, gluconic acid, succinic acid, arabic acid, xylonic acid, or mucic acid.

The polysaccharide derivative having a grafted o-dihydroxyl group in step (1) can also be prepared by the following method: providing an aqueous solution comprising 0.5% to 15% by weight of carboxypolysaccharide and an aqueous solution comprising 0.2% to 30% by weight of a small molecule compound with an amino group and an o-dihydroxyl group respectively, adding the carboxyl activating agents EDC and NHS into the carboxypolysaccharide solution, adjusting pH to 3.5 to 7, stirring the resulting solution for 0.5 to 3 h; adding the small molecule compound solution into the carboxypolysaccharide solution to form a reaction system, stirring the reaction system and adjusting the pH thereof to 7 to 9, and stirring the reaction system to react for 24 h; performing dialysis of the reaction product and freeze drying, or precipitating the reaction product with ethanol, then washing, precipitating, and drying the reaction product.

The small molecule compound with an amino group and an o-dihydroxyl group in step (1) includes aminopropylene glycol or aminoglucose.

The polysaccharide derivative with a primary amino group on a side chain in step (2) is prepared by the following steps: preparing an aqueous solution comprising 0.5% to 15% by weight of carboxypolysaccharide, and preparing an aqueous solution comprising 0.2% to 30% by weight of a small molecule compound with two or more primary amino groups, adding the carboxyl activating agents EDC and NHS into the carboxypolysaccharide solution, adjusting pH to 3.5 to 7, stirring the resulting solution for 0.5 to 3 h; adding the small molecule compound solution into the carboxypolysaccharide solution to form a reaction system, adjusting the pH to 7-9, and stirring the reaction system to react for 24 h; performing dialysis of the reaction product and freeze drying, or precipitating the reaction product with ethanol, then washing, precipitating, and drying the reaction product.

The molar ratio of the carboxyl groups to the carboxyl activating agent EDC and to the carboxyl activating agent NHS is 1: (0.5 to 6): (0.5 to 6).

The oxidizing agent includes sodium periodate or potassium periodate.

In a specific embodiment, the biological polysaccharide hydrogel of the invention is characterized by being formed by the reaction of two agents. The first agent is a solution of a polysaccharide derivative with an aldehyde group on a side chain, and the second agent is a solution of a polysaccharide derivative with a primary amino group on a main chain or on a side chain, or a solution of a small molecule compound with two or more primary amino groups.

The first agent is a solution of a polysaccharide derivative with an aldehyde group on a side chain. The percentage of saccharide units with side-chain aldehyde groups in the polysaccharide derivative accounts for 1% to 100% based on the total saccharide units therein, and the mass percentage concentration of the polysaccharide derivative in the solution is 0.5% to 15%. The second agent is a solution of a polysaccharide derivative with a primary amino group on a main chain or on a side chain. The percentage of saccharide units having a main-chain primary amino group or having a side-chain primary amino group in the polysaccharide derivative accounts for 1 % to 100% based on the total saccharide units therein, and the mass percentage concentration of the polysaccharide derivative in the solution is 0.5% to 15%. The second agent is a solution of a small molecule compound with two or more primary amino groups, and the mass percentage concentration of the small molecule compound in the solution is 0.2% to 30%. The solvents of the first and second agents are sterilized water, physiological saline, physiological balanced solution, or other aqueous medical liquids known to those skilled in the art, such as glucose solution, etc.

The first agent, a polysaccharide derivative with an aldehyde group on a side chain, is characterized by being an aminopolysaccharide derivative with an aldehyde group on a side chain or a carboxypolysaccharide derivative with an aldehyde group on a side chain. The aminopolysaccharide derivative with an aldehyde group on a side chain or the carboxypolysaccharide derivative with an aldehyde group on a side chain is formed by oxidation of an aminopolysaccharide derivative with a grafted o-dihydroxyl group on a side chain or a carboxypolysaccharide derivative with a grafted o-dihydroxyl group on a side chain. The aminopolysaccharides include, but are not limited to, hydroxyethyl chitosan, hydroxypropyl chitosan, hydroxybutyl chitosan, carboxymethyl chitosan, carboxyethyl chitosan, and other chitosan derivatives. The carboxypolysaccharides include, but are not limited to, chitosan derivatives with a carboxyl group, chitin derivatives with a carboxyl group, hyaluronic acid with a carboxyl group or hyaluronic acid derivatives with a carboxyl group, and chondroitin sulfate, heparin, sodium alginate. The chitosan derivatives with a carboxyl group include carboxymethyl chitosan, carboxyethyl chitosan, succinyl chitosan and other chitosan derivatives with a carboxyl group. The chitin derivatives with a carboxyl group include carboxymethyl chitin, carboxyethyl chitin and other chitin derivatives with a carboxyl group. The hyaluronic acid with a carboxyl group and hyaluronic acid derivatives with a carboxyl group include hyaluronic acid, hydroxyethyl hyaluronic acid, hydroxypropyl hyaluronic acid, acetyl hyaluronic acid, butyryl hyaluronic acid, aminopropionic hyaluronic acid, aminobutyric hyaluronic acid, and other hyaluronic acid derivatives. The aminopolysaccharide derivative with an aldehyde group on a side chain is characterized by having an o-dihydroxyl group grafted onto the amino group of the aminopolysaccharide, thus forming an o-dihydroxyl group on a side chain, which are further oxidized to form an aldehyde group, i.e., forming an aminopolysaccharide derivative with an aldehyde group on a side chain. The carboxypolysaccharide derivative with an aldehyde group on a side chain is characterized by having an o-dihydroxyl group grafted onto the carboxyl group of the carboxypolysaccharide, thus forming an o-dihydroxyl group on a side chain, which are further oxidized to form an aldehyde group, i.e., forming a carboxypolysaccharide derivative with an aldehyde group on a side chain. The grafting rate of o-dihydroxyl groups on the aminopolysaccharide or the carboxypolysaccharide can be adjusted by controlling the ratio of the materials and reaction conditions, and theoretically can be between 1% and 100%.

The side-chain o-dihydroxyl structure refers to an o-dihydroxyl group grafted onto the amino group of an aminopolysaccharide or onto the carboxyl group of a carboxypolysaccharide. The compound providing the o-dihydroxyl structure for an aminopolysaccharide is a small molecule compound that simultaneously has a carboxyl group and an o-dihydroxyl group, including but not limited to 2,3-dihydroxypropanoic acid, 2,3,4-trihydroxybutanoic acid, gluconic acid, succinic acid, arabic acid, xylonic acid, mucic acid, and other compounds that simultaneously have a carboxyl group and an o-dihydroxyl group. The aminopolysaccharide and the compound that simultaneously has a carboxyl group and an o-dihydroxyl group undergo a condensation reaction between the amino group and the carboxyl group, causing the aminopolysaccharide to be grafted with an o-dihydroxyl group on a side chain, forming a polysaccharide derivative with an o-dihydroxyl group on a side chain. The compound providing the o-dihydroxyl structure for the carboxypolysaccharides is a small molecule compound that simultaneously has an amino group and an o-dihydroxyl group, including but not limited to aminopropylene glycol, aminoglucose, and other compounds that simultaneously have an amino group and an o-dihydroxyl group. The carboxypolysaccharide and the compound that simultaneously has an amino group and an o-dihydroxyl group undergo a condensation reaction between the carboxyl group and the amino group, causing the carboxypolysaccharide to be grafted with an o-dihydroxyl group on a side chain, forming a polysaccharide derivative with an o-dihydroxyl group on a side chain. The polysaccharide derivative with a grafted o-dihydroxyl group on a side chain is further oxidized to form a polysaccharide derivative with an aldehyde group on a side chain. The oxidation of the side-chain o-dihydroxyl groups can yield different contents of aldehyde groups theoretically ranging from 1% to 100% by controlling the conditions including concentration of the oxidizing agent, time, and temperature, etc.

The second agent is a polysaccharide derivative with a primary amino group on a main chain or on a side chain, or a small molecule compound with two or more primary amino groups. The polysaccharide derivative with a primary amino group on a main chain is characterized by having both a primary amino group on a main chain and a side-chain grafted structure, including but not limited to hydroxyethyl chitosan, hydroxypropyl chitosan, hydroxybutyl chitosan, carboxymethyl chitosan, carboxyethyl chitosan, and other chitosan derivatives. The polysaccharide derivative with a primary amino group on a side chain is characterized by having a carboxyl group on a main chain and a primary amino group on a side chain, which is formed by the condensation of a carboxyl group of a carboxypolysaccharide with one amino group of a small molecule compound having two or more primary amino groups. The carboxypolysaccharides include, but are not limited to, chitosan derivatives with a carboxyl group, chitin derivatives with a carboxyl group, hyaluronic acid with a carboxyl group and hyaluronic acid derivatives with a carboxyl group, and chondroitin sulfate, heparin, sodium alginate, same as the carboxypolysaccharides mentioned in the first agent. The small molecule compound with two or more primary amino groups is characterized by being an aliphatic or aromatic small molecule compound with two or more primary amino groups, including but not limited to ethylenediamine, propylenediamine, butylenediamine, aminobutyramide, glutamine, propionyl glutamine, lysine and other basic amino acids, small molecule compounds with a dihydrazide structure, diamino benzoic acid, diamino phenylacetic acid. The small molecule compound with a dihydrazide structure includes carbohydrazide, oxalyl dihydrazide, propanedioyl dihydrazide, butanedioyl dihydrazide, adipic dihydrazide, sebacic dihydrazide, and other compounds having a dihydrazide structure. The grafting rate of primary amino groups on the carboxypolysaccharides, theoretically ranging from 1 to 100%, can be adjusted by controlling the ratio of the raw materials and reaction conditions.

The biological polysaccharide hydrogel is formed by mixing a first solution, which is a polysaccharide derivative with an aldehyde group on a side chain, with a second solution, which is a polysaccharide derivative with a primary amino group on a main chain or on a side chain, or a small molecule compound with two or more primary amino groups. The aldehyde group of the first agent react with the primary amino group of the second agent to form a hydrogel with high transparency, viscoelasticity, and biocompatibility.

The preparation method of the above-mentioned biological polysaccharide hydrogel is characterized by comprising the preparation of the first agent and the second agent, and mixing of the two agents. The specific preparation method comprises the following steps:

### Preparation of the first agent:

(1) Preparation of a polysaccharide derivative having a grafted o-dihydroxyl group: 1) Preparing an aqueous solution of an aminopolysaccharide with a mass percentage concentration of 0.5 to 15% and an aqueous solution of a small molecule compound having a carboxyl group and an o-dihydroxyl group with a mass percentage concentration of 0.2 to 30%, respectively. Adding the carboxyl activating agents 1-(3-dimethylaminopropyl)-3-ethylcarbodiimide hydrochloride (EDC) and N-hydroxysuccinimide (NHS) to the small molecule compound solution, adjusting the pH to 3.5 to 7, and stirring for 0.5 to 3 hours. Then, adding the small molecule compound solution to the aminopolysaccharide solution, stirring the resulting solution and adjusting the pH of the to 7 to 9, and stirring to react for 24 hours; or 2) Preparing an aqueous solution of a carboxypolysaccharide with a mass percentage concentration of 0.5 to 15% and an aqueous solution of a small molecule compound having an amino group and an o-dihydroxyl group with a mass percentage concentration of 0.2 to 30%, respectively. Adding the carboxyl activating agents EDC and NHS to the carboxypolysaccharide solution, adjusting the pH to 3.5 to 7, and stirring for 0.5 to 3 hours. Then, adding the small molecule compound solution to the carboxypolysaccharide solution, stirring the resulting solution and adjusting the pH to 7 to 9, and stirring to react for 24 hours. Finally, performing dialysis of the reaction product and freeze-drying, or precipitating the reaction product with ethanol, washing, precipitating, and drying, to obtain a polysaccharide derivative having a grafted o-dihydroxyl group, and measuring the grafting rate of the o-dihydroxyl groups of the polysaccharide derivative;
(2) Preparation of a polysaccharide derivative with an aldehyde group on a side chain: Preparing an aqueous solution of a polysaccharide derivative having a grafted o-dihydroxyl group at a mass percentage concentration of 0.5 to 15%, adding an oxidizing agent with the molar ratio of o-dihydroxyl groups to the oxidizing agent being 1: (0.3 to 3), stirring the resulting solution to react at room temperature or lower temperature under dark condition for 2 minutes to 5 hours, adding an ethylene glycol solution to terminate the reaction for 0.5 to 2 hours, performing dialysis of the reaction product and freeze-drying, or precipitating the reaction product with ethanol, washing, precipitating, and drying, to obtain a polysaccharide derivative with an aldehyde group on a side chain, and measuring the mole percentage content of the aldehyde groups;
(3) Preparation of a solution of a polysaccharide derivative with an aldehyde group on a side chain: Preparing a sterilized solution of a polysaccharide derivative with an aldehyde group on a side chain at a mass percentage concentration of 0.5 to 15%, and adjusting pH to 3.5 to 7.

### Preparation of the second agent:

(1) Solution of a small molecule compound with two or more primary amino groups: Preparing a sterilized solution of a small molecule compound with two or more primary amino groups at a mass percentage concentration of 0.2% to 30%, adjusting pH to 7 to 9, and measuring the mole percentage content of primary amino groups;
or (2) Solution of a polysaccharide derivative with a primary amino group on a main chain: Preparing a sterilized solution of a polysaccharide derivative with primary amino groups on a main chain at a mass percentage concentration of 0.5% to 15%, adjusting pH to 7 to 9, and measuring the mole percentage content of primary amino groups;
or (3) Solution of a polysaccharide derivative with a primary amino group on a side chain: Preparing an aqueous solution of a carboxypolysaccharide with a mass percentage concentration of 0.5% to 15%, preparing an aqueous solution of a small molecule compound with two or more primary amino groups with a mass percentage concentration of 0.2% to 30%, adding carboxyl activating agents EDC and NHS to the carboxypolysaccharide solution, adjusting pH to 3.5 to 7, and stirring for 0.5 to 3 hours. Then, adding the small molecule compound solution to the carboxypolysaccharide solution, adjusting the pH to 7 to 9, and stirring the resulting solution to react for 24 hours. Finally, performing dialysis of the reaction product and freeze-drying, or precipitating the reaction product with ethanol, washing, precipitating, and drying, to obtain a polysaccharide derivative with a primary amino group on a side chain. Preparing a sterilized solution of the polysaccharide derivative with a primary amino group on a side chain at a mass percentage concentration of 0.5% to 15%, adjusting pH to 7 to 9, and measuring the mole percentage content of primary amino groups.

### Mixing of the first and second agents:

Charging the first agent, a solution of a polysaccharide derivative with an aldehyde group on a side chain, and the second agent, a solution of a small molecule compound with two or more primary amino groups, or a solution of a polysaccharide derivative with a primary amino group on a main chain or on a side chain, separately into tube A and tube B of a sterilized dual syringe, and mixing them by pushing them out from the syringe. Mixing the first and second agents to form a transparent biological polysaccharide hydrogel.

In the above-mentioned preparation method of the biological polysaccharide hydrogel, the molar ratio of the carboxyl groups in the small molecule compound with a carboxyl group and an o-dihydroxyl group, or the carboxyl groups in the carboxypolysaccharide, to the carboxyl activating agents EDC and NHS is 1: (0.5 to 6): (0.5 to 6). The oxidizing agents include, but are not limited to, sodium periodate and potassium periodate.

In another aspect, the invention relates to the use of the aforementioned biological polysaccharide hydrogel in the preparation of intraocular vitreous substitutes.

In another aspect, the invention also relates to the aforementioned biological polysaccharide hydrogel, which is used as intraocular vitreous substitutes.

In yet another aspect, the invention relates to an intraocular vitreous substitute, which includes a biological polysaccharide hydrogel formed by the reaction of a first solution and a second solution. The first solution is a solution of a polysaccharide derivative with an aldehyde group on a side chain, and the second solution is a solution of a polysaccharide derivative with a primary amino group on a main chain or on a side chain, or a solution of a small molecule compound with two or more primary amino groups.

The first solution contains a polysaccharide derivative with an aldehyde group on a side chain at a mass percentage concentration of 0.5% to 15%. The second solution contains a polysaccharide derivative with a primary amino group on a main chain or on a side chain at a mass percentage concentration of 0.5% to 15%, or a small molecule compound with two or more primary amino groups at a mass percentage concentration of 0.2% to 30%. The solvents for the first and second solutions are selected from sterilized water, physiological saline, balanced physiological solution, and glucose solution.

The polysaccharide derivative with an aldehyde group on a side chain in the first solution of this invention is an aminopolysaccharide derivative with an aldehyde group on a side chain or a carboxypolysaccharide derivative with an aldehyde group on a side chain. The aminopolysaccharide derivative with an aldehyde group on a side chain is an amino olysaccharide derivative whose amino group is modified to form a side chain having an aldehyde group. The carboxypolysaccharide derivative with an aldehyde group on a side chain is a carboxypolysaccharide derivative whose carboxyl group is modified to form a side chain having an aldehyde group. The aminopolysaccharide derivative with an aldehyde group on a side chain or the carboxypolysaccharide derivative with an aldehyde group on a side chain is formed by oxidation of an aminopolysaccharide whose amino group has been modified into a side chain having an o-dihydroxy group, or a carboxypolysaccharide whose carboxyl group has been modified into a side chain having an o-dihydroxy group.

The amino polysaccharide derivative in the first solution of this invention includes hydroxyethyl chitosan, hydroxypropyl chitosan, hydroxybutyl chitosan, carboxymethyl chitosan, or carboxyethyl chitosan. The carboxy polysaccharide derivative includes chitosan derivatives with a carboxyl group, chitin derivatives with a carboxyl group, hyaluronic acid or hyaluronic acid derivatives with a carboxyl group, chondroitin sulfate, heparin, or sodium alginate.

The small molecule compound with two or more primary amino groups in this invention is an aliphatic or aromatic small molecule compound with two or more primary amino groups, including ethylenediamine, propylenediamine, butylenediamine, aminobutyramide, glutamine, propionyl glutamine, lysine or other basic amino acids, a small molecule compound with a dihydrazide structure, diamino benzoic acid, diamino phenylacetic acid.

The small molecule compound with a dihydrazide structure includes carbohydrazide, oxalyl dihydrazide, propanedioyl dihydrazide, butanedioyl dihydrazide, adipic dihydrazide or sebacic dihydrazide.

The polysaccharide derivative with primary amino groups on a main chain in the second solution of this invention is an amino polysaccharide derivative whose main chain has primary amino groups and also has side-chain grafted structures. The polysaccharide derivative with primary amino groups on the side chains is a polysaccharide derivative whose main chain has a carboxyl structure and the side chains have primary amino groups. The polysaccharide derivative with primary amino groups on the side chains is formed by the condensation of a carboxyl group of a carboxy polysaccharide with one amino group of a small molecule compound with two or more primary amino groups. In yet another aspect, the invention involves a biological polysaccharide hydrogel, which is used as an intraocular vitreous substitute. This biological polysaccharide hydrogel is formed by the reaction of a first solution and a second solution. The first solution is a solution of a polysaccharide derivative with an aldehyde group on a side chain, and the second solution is a solution of a polysaccharide derivative with a primary amino group on a main chain or on a side chain, or a solution of a small molecule compound with two or more primary amino groups.

The first solution in this invention contains a polysaccharide derivative with an aldehyde group on a side chain at a mass percentage concentration of 0.5% to 15%. The second solution contains a polysaccharide derivative with a primary amino group on a main chain or on a side chain at a mass percentage concentration of 0.5% to 15%, or a small molecule compound with two or more primary amino groups at a mass percentage concentration of 0.2% to 30%. The solvents for the first and second solutions are selected from sterile water, physiological saline, balanced physiological solution, and glucose solution.

The polysaccharide derivative with an aldehyde group on a side chain in the first solution of this invention is an amino polysaccharide derivative with an aldehyde group on a side chain or a carboxypolysaccharide derivative with an aldehyde group on a side chain. The amino polysaccharide derivative with an aldehyde group on a side chain is an aminopolysaccharide derivative whose amino group has been modified to form an aldehyde group on a side chain. The carboxypolysaccharide derivative with an aldehyde group on a side chain is a carboxypolysaccharide derivative whose carboxyl group has been modified to form an aldehyde group on a side chain. The aminopolysaccharide derivative with an aldehyde group on a side chain or the carboxypolysaccharide derivative with an aldehyde group on a side chain is formed by oxidation of an aminopolysaccharide whose amino group has been modified to form an o-dihydroxy group on a side chain, or a carboxypolysaccharide whose carboxyl group has been modified to form an o-dihydroxy group on a side chain.

The amino polysaccharide derivative in the first solution of this invention includes hydroxyethyl chitosan, hydroxypropyl chitosan, hydroxybutyl chitosan, carboxymethyl chitosan, or carboxyethyl chitosan. The carboxypolysaccharide derivative includes chitosan derivatives with a carboxyl group, chitin derivatives with a carboxyl group, hyaluronic acid or hyaluronic acid derivatives with a carboxyl group, chondroitin sulfate, heparin, or sodium alginate.

The small molecule compound with two or more primary amino groups in this invention is an aliphatic or aromatic small molecule compound with two or more primary amino groups, including ethylenediamine, propylenediamine, butylenediamine, aminobutyramide, glutamine, propionyl glutamine, lysine or other basic amino acids, a small molecule compound with a dihydrazide structure, diamino benzoic acid, diamino phenylacetic acid.

The small molecule compound with a dihydrazide structure includes carbohydrazide, oxalyl dihydrazide, propanedioyl dihydrazide, butanedioyl dihydrazide, adipic dihydrazide or sebacic dihydrazide.

The polysaccharide derivative with primary amino groups on the main chain in the second solution of this invention is an amino polysaccharide derivative whose main chain has primary amino groups and also has grafted groups on the side chains. The polysaccharide derivative with primary amino groups on the side chains is a polysaccharide derivative whose main chain has a carboxyl structure and the side chains have primary amino groups. The polysaccharide derivative with primary amino groups on the side chains is formed by the condensation of a carboxyl group of a carboxy polysaccharide with one amino group of a small molecule compound with two or more primary amino groups.

The preparation method of the biological polysaccharide hydrogel comprised in the intraocular vitreous substitute of this invention includes the following steps:
(1) providing a first solution, which is a sterilized solution comprising 0.5% to 15% by weight of a polysaccharide derivative with an aldehyde group on a side chain, and adjusting the pH of the first solution to 3.5 to 7, wherein the polysaccharide derivative with an aldehyde group on a side chain is formed by the reaction of an aqueous solution of a polysaccharide derivative having a grafted o-dihydroxyl group with an oxidizing agent;
(2) providing a second solution selected from the group consisting of:
   i) a sterilized solution comprising 0.2% to 30% by weight of a small molecule compound with two or more primary amino groups;
   ii) a sterilized solution comprising 0.5% to 15% by weight of a polysaccharide derivative with a primary amino group on a main chain;
   iii) a sterilized solution comprising 0.5% to 15% by weight of a polysaccharide derivative with a primary amino group on a side chain;
   and adjusting the pH of the second solution to 7 to 9, and measuring the mole percentage content of primary amino groups therein;
(3) mixing the first solution with the second solution: adding the first solution obtained in step (1) and the second solution obtained in step (2) respectively into tube A and tube B of a sterilized dual syringe, pushing them out from the syringe for mixing and forming a transparent biological polysaccharide hydrogel by mixing the first solution and the second solution.

The polysaccharide derivative with an aldehyde group on the side chain in step (1) is prepared by the following method: providing an aqueous solution comprising 0.5% to 15% by weight of a polysaccharide derivative having a grafted o-dihydroxyl group, adding the oxidizing agent, wherein the molar ratio of the o-dihydroxyl group to the oxidizing agent is 1: (0.3 to 3), stirring the reaction mixture at room temperature or lower temperature and under dark condition for 2 minutes to 5 hours, adding an ethylene glycol solution to terminate the reaction for 0.5 to 2 hours, and performing dialysis of the reaction product and freeze drying, or precipitating with ethanol, washing, precipitating, and drying.

The polysaccharide derivative having a grafted o-dihydroxyl group in step (1) is prepared by the following method: providing an aqueous solution comprising 0.5% to 15% by weight of an aminopolysaccharide and an aqueous solution comprising 0.2% to 30% by weight of a small molecule compound with a carboxyl group and an o-dihydroxyl group respectively, adding the carboxyl activating agents 1-(3-dimethylaminopropyl)-3-ethylcarbodiimide hydrochloride and N-hydroxysuccinimide to the small molecule compound solution, and adjusting the pH to 3.5 to 7, stirring the resulting solution to react for 0.5 to 3 hours; adding the obtained small molecule compound solution to the aminopolysaccharide solution to form a reaction system, stirring and adjusting the pH of the reaction system to 7 to 9, and stirring the reaction system to react for 24 hours.

The small molecule compound with carboxyl and o-dihydroxyl groups in step (1) includes 2,3-dihydroxypropanoic acid, 2,3,4-trihydroxybutanoic acid, gluconic acid, succinic acid, arabic acid, xylonic acid, or mucic acid.

The polysaccharide derivative having a grafted o-dihydroxyl group in step (1) can also be prepared by the following method: providing an aqueous solution comprising 0.5% to 15% by weight of carboxypolysaccharide and an aqueous solution comprising 0.2% to 30% by weight of a small molecule compound with an amino group and an o-dihydroxyl group respectively, adding the carboxyl activating agents EDC and NHS into the carboxypolysaccharide solution, adjusting pH to 3.5 to 7, stirring the resulting solution for 0.5 to 3 h; adding the small molecule compound solution into the carboxypolysaccharide solution to form a reaction system, stirring the reaction system and adjusting the pH thereof to 7 to 9, and stirring the reaction system to react for 24 h; performing dialysis of the reaction product and freeze drying, or precipitating the reaction product with ethanol, then washing, precipitating, and drying.

The small molecule compound with an amino group and an o-dihydroxyl group in step (1) includes aminopropylene glycol or aminoglucose.

The polysaccharide derivative with a primary amino group on a side chain in step (2) is prepared by the following steps: preparing an aqueous solution comprising 0.5% to 15% by weight of carboxypolysaccharide, and preparing an aqueous solution comprising 0.2% to 30% by weight of a small molecule compound with two or more primary amino groups, adding the carboxyl activating agents EDC and NHS into the carboxypolysaccharide solution, adjusting pH to 3.5 to 7, stirring the resulting solution for 0.5 to 3 h; adding the small molecule compound solution into the carboxypolysaccharide solution to form a reaction system, adjusting the pH to 7-9, and stirring the reaction system to react for 24 h; performing dialysis of the reaction product and freeze drying, or precipitating the reaction product with ethanol, then washing, precipitating, and drying.

The molar ratio of the carboxyl groups to the carboxyl activating agents EDC and NHS is 1: (0.5 to 6): (0.5 to 6).

The oxidizing agent includes sodium periodate or potassium periodate.

The raw materials for the biological polysaccharide hydrogel of this invention are selected for their good biocompatibility. The prepared polysaccharide hydrogel has excellent light transmissivity, viscoelasticity, and tissue non-toxicity. By having side-chain aldehyde groups and maintaining the integrity of the polysaccharide molecule structure of the aldehyde donor, and through further cross-linking of the amino donor polysaccharide molecule with side-chain grafted structures and small molecule compounds with two or more primary amino groups, the specific recognition of polysaccharides by bio-enzymes is reduced due to the side-chain grafted structure, the maintenance of the integrity of the polysaccharide molecule structure, and the cross-linking of small molecule compounds. This enhances the biodegradation resistance of the cross-linked polysaccharide hydrogel, extends the effective pressure-holding time of the hydrogel on the retina, and achieves the purpose of being suitable for use as a vitreous substitute. The biological polysaccharide hydrogel of this invention as a vitreous substitute can maintain the shape of the eyeball and keep the retina in place under pressure, can improve the shock absorption function, and has good self-healing properties.

### BRIEF DESCRIPTION OF THE DRAWINGS

Figure 1 shows the eye pressure tests of an operated eye and a normal eye.
Figure 2 shows a slit lamp observation image of an operated eye.
Figure 3 shows a fundus photography observation image of an operated eye.
Figure 4 shows a B-ultrasound examination image of an operated eye.

### DETAILED DESCRIPTION OF THE INVENTION

The invention will be further specified with reference to the accompanying drawings and examples.

### Example 1: Preparation of a polysaccharide derivative with a grafted o-dihydroxyl group on an amino polysaccharide

5.5 g of small molecule compound with a carboxyl and o-dihydroxyl group, 2,3,4-trihydroxybutanoic acid, was weighed and dissolved in 150 mL of deionized water. The carboxyl activating agents EDC and NHS were added at 11.4 g and 7.0 g respectively, and the mixture was stirred until dissolved and the pH of the mixture was adjusted to 4 to obtain solution 1. 4.1 g of hydroxyethyl chitosan, an aminopolysaccharide, was weighed and stirred until dissolved in 100 mL of deionized water to obtain solution 2. Solution 1 was added dropwise to solution 2 under stirring, the pH of the resulting solution was adjusted to 7.5, and the resulting solution was stirred to react at room temperature for 24 hours. After the reaction was completed, the reaction product was loaded into a dialysis bag with a molecular weight cut-off of 8000-14000 and dialyzed in deionized water for 48 hours, then the resulting solution was freeze-dried to obtain a polysaccharide derivative with a grafted o-dihydroxyl group on the aminopolysaccharide (trihydroxybutanoic acid-hydroxyethyl chitosan). The grafting rate (molar ratio) of the o-dihydroxyl groups of the polysaccharide derivative was determined to be 92%.

In the preparation of the polysaccharide derivative with a grafted o-dihydroxyl group on the aminopolysaccharide in Example 1, by increasing the molar ratio of the small molecule compound like trihydroxybutanoic acid that simultaneously has a carboxyl group and an o-dihydroxyl group, the polysaccharide with an amino group can be grafted with a high proportion of o-dihydroxyl groups. The prepared polysaccharide derivative with a grafted o-dihydroxyl group on the aminopolysaccharide has a high o-dihydroxyl grafting rate, which can reduce the molar ratio of primary amino groups in the aminopolysaccharide, and thus a high proportion of aldehyde groups can be obtained by oxidation.

### Example 2: Preparation of a polysaccharide derivative with a grafted o-dihydroxyl group on a carboxypolysaccharide

4 g of Sodium hyaluronate, a carboxypolysaccharide, was weighed and stirred until dissolved in 200 mL of deionized water. The carboxyl activating agents EDC and NHS were added at 2.87 g and 1.73 g repectively, and the mixture was stirred at room temperature until dissolved and the pH was adjusted to 5 to obtain solution 1. 1.2 mL of aminopropylene glycol, a small molecule compound with an amino group and an o-dihydroxyl group, was measured out and diluted with 2 mL of deionized water to obtain solution 2. Solution 2 was added to solution 1 to obtain a reaction system, and the pH of the reaction system was adjusted to 8.5, and the reaction system was stirred for 24 hours. After the reaction was completed, the reaction product was precipitated with ethanol, washed, precipitated again, and dried to obtain a polysaccharide derivative with a grafted o-dihydroxyl group on the carboxypolysaccharide (aminopropylene glycol-hyaluronic acid), and the grafting rate of the o-dihydroxyl groups of the polysaccharide derivative was determined to be 88%.

In the preparation of the polysaccharide derivative with a grafted o-dihydroxyl group on the carboxypolysaccharide in Example 2, by adjusting the molar ratio of the small molecule compound like aminopropylene glycol that simultaneously has an amino group and an o-dihydroxyl group to the carboxypolysaccharide, the polysaccharide with carboxyl groups can be grafted with o-dihydroxyl groups at different proportions, forming polysaccharide derivatives with different molar ratios of side-chain o-dihydroxyl groups, and different proportions of o-dihydroxyl groups can be oxidized to obtain different proportions of aldehyde groups.

### Example 3: Preparation of polysaccharide derivative 1 with an aldehyde group on the side chain

3.0 g of the polysaccharide derivative with a grafted o-dihydroxyl group on an aminopolysaccharide prepared in Example 1 was weighed and stirred until dissolved in 100 mL of deionized water to obtain solution 1. And 2.0 g of sodium periodate, an oxidizing agent, was weighed and stirred until dissolved in 40 mL of deionized water to obtain solution 2. Solution 2 was added dropwise to solution 1 under stirring, and the oxidation reaction was carried out at room temperature for 10 to 20 minutes. After the reaction was completed, 3 mL of ethylene glycol, a terminating agent, was added to terminate the reaction, and the stirring was continued for 1 hour. The reaction product was precipitated with ethanol, washed, precipitated again, and dried to obtain an aminopolysaccharide derivative with an aldehyde group on a side chain (oxidized trihydroxybutanoic acid-hydroxyethyl chitosan), and the mole percentage content of the aldehyde groups was determined to be 65%.

### Example 4: Preparation of polysaccharide derivative 2 with an aldehyde group on a side chain

4.0 g of the polysaccharide derivative with a grafted o-dihydroxyl group on a carboxypolysaccharide prepared in Example 2 was weighed and stirred until dissolved in 200 mL of deionized water to obtain solution 1. And 1.5 g of sodium periodate, an oxidizing agent, was weighed and stirred until dissolved in 40 mL of deionized water to obtain solution 2. Solution 2 was added dropwise to solution 1 under stirring, and the oxidation reaction was carried out at 8 to 10°C for 40 minutes to 2 hours. After the reaction was completed, 4 mL of ethylene glycol was added as a terminating agent to terminate the reaction, and the stirring was continued for 1 hour. The reaction product was dialyzed in deionized water for 48 hours in a dialysis bag with a molecular weight cut-off of 8000-14000, then the resulting solution was freeze-dried to obtain a carboxypolysaccharide derivative with an aldehyde group on a side chain (oxidized aminopropylene glycol-hyaluronic acid), and the mole percentage content of the aldehyde groups was determined to be 46%.

In the preparation of polysaccharide derivatives with an aldehyde group on a side chain in Example 3 and Example 4, the polysaccharide derivatives with a grafted o-dihydroxyl group on a side chain were oxidized by the oxidizing agent sodium periodate. The reaction temperature was adjusted, and the ratio of the materials was controlled and the reaction time was controlled to be from several minutes to 5 hours. The oxidizing agent preferentially oxidized the o-dihydroxyl group on a side chain of the polysaccharide to form an aldehyde group, without oxidizing the main chain of the polysaccharide, thereby ensuring that the main chain of the polysaccharide molecule was not ring-opened and degraded, maintaining the integrity of the main chain of polysaccharide. The oxidizing agents used include but are not limited to sodium periodate and potassium periodate. By controlling the amount of oxidizing agent added, controlling the oxidation reaction time, and controlling the reaction temperature, different molar ratios of aldehyde groups on the side chains were obtained, and the main chain of the polysaccharide was not ring-opened by oxidation. Based on total saccharide units of the prepared polysaccharide derivative with an aldehyde group on a side chain, the molar percentage of the side-chain aldehyde units of the prepared polysaccharide derivative with an aldehyde group on a side chain can be ≥1%, such as 5%, 10%, 20%, 30%, 40%, 50%, 60%, etc., or can be close to 100%.

### Example 5: Preparation of polysaccharide derivative 1 with a primary amino group on a side chain

4.5 g of carboxymethyl chitosan, a carboxypolysaccharide, was weighed and stirred until dissolved in 150 mL of deionized water. The carboxyl activating agents EDC and NHS were added at 3.0 g and 1.8 g respectively, and the mixture was stirred for 1 hour, and the pH was adjusted to 6 to obtain solution 1. And 2.0 g of carbohydrazide, a small molecule compound with two or more primary amino groups, was weighed and stirred until dissolved in 30 mL of deionized water to obtain solution 2. Solution 2 was added dropwise to solution 1 under stirring to obtain a reaction system, the pH of the reaction system was adjusted to 8, and then the reaction was continued for 24 hours. After the reaction was completed, the reaction product was precipitated with ethanol, washed, precipitated again, and dried to obtain a polysaccharide derivative with a primary amino group on a side chain (carbohydrazide-carboxymethyl chitosan), and the grafting rate of primary amino groups was determined to be 62%.

### Example 6: Preparation of polysaccharide derivative 2 with a primary amino group on a side chain

4 g of Sodium hyaluronate, a carboxypolysaccharide, was weighed and stirred until dissolved in 100 mL of deionized water. The carboxyl activating agents EDC and NHS were added at 1.91 g and 1.15 g respectively, and the mixture was stirred for 1 hour, and the pH was adjusted to 6 to obtain solution 1. And 2.9 g of glutamine, a small molecule compound with two or more primary amino groups, was weighed and stirred until dissolved in 20 mL of deionized water to obtain solution 2. Solution 2 was added dropwise to solution 1 under stirring to obtain a reaction system, the pH of the reaction system was adjusted to 7, and the reaction was continued for 24 hours. After the reaction was completed, the reaction product was precipitated with ethanol, washed, precipitated, and dried to obtain a polysaccharide derivative with a primary amino group on a side chain (glutamine-hyaluronic acid), and the grafting rate of primary amino groups was determined to be 43%.

In the preparation of the polysaccharide derivatives with a primary amino group on a side chain in Example 5 and Example 6, by controlling the molar ratio of small molecule compounds like carbohydrazide and glutamine that have two or more primary amino groups, and by controlling the reaction time, different molar ratios of grafted primary amino structures on the side chains were obtained. Based on to the total saccharide units of the prepared polysaccharide derivative with a primary amino group on a side chain, the molar percentage of the primary amino units on the side chains of the prepared polysaccharide derivative with a primary amino group on a side chain can be ≥1%, such as 5%, 10%, 20%, 30%, 40%, 50%, 60%, etc., or can be close to 100%.

### Example 7: Preparation of polysaccharide hydrogel 1

First agent: 1.0 g of sterilized sample of the aminopolysaccharide derivative with an aldehyde group on a side chain as prepared in Example 3 was weighed and dissolved in 10 mL of sterilized physiological saline to prepare a solution with a mass percentage concentration of 10%. The pH was adjusted to 6.

Second agent: 0.05 g of sterilized sample of succinic dihydrazide, a small molecule compound with two or more primary amino groups, was weighed and dissolved in 10 mL of sterilized physiological saline to prepare a solution with a mass percentage concentration of 0.5%. The pH was adjusted to 7.5.

The first agent and the second agent were charged separately into tube A and tube B of a sterilized dual syringe. The first agent and the second agent were mixed by pushing them out from the syringe to form a transparent polysaccharide hydrogel 1. The gelation time was about 2 minutes. The formed polysaccharide hydrogel was viscous and had good resilience when pressed.

### Example 8: Preparation of Polysaccharide Hydrogel 2

First agent: 0.3 g of the aminopolysaccharide derivative with an aldehyde group on a side chain as prepared in Example 3 was weighed and dissolved in 10 mL of sterilized physiological saline to prepare a solution with a mass percent concentration of 3%. The pH was adjusted to 5.

Second agent: 0.2 g of the polysaccharide derivative with a primary amino group on a side chain (glutamine-hyaluronic acid) prepared in Example 6 was weighed and dissolved in 10 mL of sterilized saline to prepare a solution with a mass percent concentration of 2%. The pH was adjusted to 8.

The first and second agents were respectively charged into tube A and tube B of a sterilized dual syringe. The first and second agents were mixed by pushing them out from the syringe, forming a transparent polysaccharide hydrogel 2. The gelation time was about 5 minutes. The formed polysaccharide hydrogel was viscous and had good resilience when pressed.

### Example 9: Preparation of Polysaccharide Hydrogel 3

First agent: 0.3 g of the carboxylpolysaccharide derivative with an aldehyde group on a side chain as prepared in Example 4 was weighed and dissolved in 10 mL of sterilized deionized water to prepare a solution with a mass percent concentration of 4%. The pH was adjusted to 4.0.

Second agent: 0.3 g of hydroxypropyl chitosan, a polysaccharide derivative with a primary amino group on a main chain, was weighed and dissolved in 10 mL of sterilized deionized water to prepare a solution with a mass percent concentration of 5%. The pH was adjusted to 8.5.

The first and second agents were respectively charged into tube A and tube B of a sterilized dual syringe. The first and second agents were mixed by pushing them out from the syringe, forming a transparent polysaccharide hydrogel 3. The gelation time was about 6 minutes. The formed polysaccharide hydrogel was viscous and had good resilience when pressed.

### Example 10: Measurement of the transmittance of the polysaccharide hydrogels

Appropriate amounts of polysaccharide hydrogels prepared in Examples 7 to 9 were respectively taken and fully swelled in physiological saline, and then placed in a glass colorimetric dish. The absorbance (A) was measured in the visible light wavelength range of 400-800 nm, and the transmittance (%) of the hydrogel was calculated according to the formula "T (%) =10^{2-A}". The results are shown in Table 1. The transmittance of the hydrogel is related to the wavelength. The transmittance increases with the increase of the wavelength. The transmittance of all hydrogels at 400 nm is greater than 88%; when the wavelength is more than 500 nm, the transmittance of all hydrogels is greater than 90%. This indicates that the polysaccharide hydrogels prepared in the above examples have excellent transmittance.

**Table 1. Transmittance of the polysaccharide hydrogelgels**

| Wave-lengths (nm) | 400 | 500 | 600 | 700 | 800 |
|---|---|---|---|---|---|
| Transmittance of polysaccharide hydrogelgel 1 (%) | 88.5 | 90.8 | 92.4 | 93.7 | 94.8 |
| Transmittance of polysaccharide hydrogelgel 2 (%) | 90.2 | 92.8 | 94.6 | 96.7 | 97.2 |
| Transmittance of polysaccharide hydrogelgel 3 (%) | 89.7 | 91.5 | 93.2 | 95.3 | 96.4 |

### Example 11: Measurement of degradability of the polysaccharide hydrogels

The polysaccharide hydrogels prepared in Examples 7 to 9 were each gelled in a small test tube with a diameter of 6 mm. The resulting gel bars were respectively taken out and cut into gel blocks with a height of 5 mm. Two blocks of each of the three different hydrogel blocks were taken out and dried to constant weight, weighed, and the solid content of the gel blocks before degradation was calculated. A buffer solution of 10,000 units/mL lysozyme was prepared and charged into three conical flasks, with each conical flask containing 100 mL of lysozyme solution. Fifteen gel blocks of each of the three types of polysaccharide hydrogels were placed into the conical flasks containing the lysozyme solution respectively and the degradability was measured in a thermotank at 37°C. Every 10 days, 2 gel blocks were taken out and dried to constant weight, weighed, and the solid contents of the degraded gel blocks were calculated, and the degradation rate was calculated. The results are shown in Table 2. The three types of polysaccharide hydrogels were relatively stable in the first 20 days, with a degradation rate of less than 20% in 30 days and around 30% in 40 days. As the degradation time increased, the degradation rate accelerated, and the degradation rate in 50 and 60 days significantly increased, with the degradation rate reached about 70% in 60 days. The experimental results show that the polysaccharide hydrogel prepared by the present invention has good anti-degradation properties, good early stability, and a degradation time of more than 60 days. In contrast, the patent application with publication number CN 102762647 A discloses that the residual weight of the prepared hydrogel is 86.67% on the third day of in vitro degradation and is 61.02% on the 35th day. Compared with hydrogel disclosed in CN 102762647 A, the in vitro degradation time of the polysaccharide hydrogel of the present invention is much longer, thus the polysaccharide hydrogel of the present invention has a longer pressure-holding time to hold the retina in place.

**Table 2. Degradation rate of the polysaccharide hydrogelgels**

| Time (days) | 10 | 20 | 30 | 40 | 50 | 60 |
|---|---|---|---|---|---|---|
| Degradation rate of polysaccharide hydrogelgel 1 (%) | 3.2% | 7.3% | 16.5% | 28.7% | 50.6% | 65.3% |
| Degradation rate of polysaccharide hydrogelgel 2 (%) | 4.8% | 9.1% | 18.8% | 32.2% | 58.2% | 73.7% |
| Degradation rate of polysaccharide hydrogelgel 3 (%) | 3.5% | 8.8% | 17.9% | 30.7% | 53.8% | 70.2% |

### Example 12: Use of the polysaccharide hydrogels as intraocular vitreous substitutes

Three female New Zealand rabbits, weighing 2.5 to 3 kg, were used as experimental animals. The left eyes of the rabbits were the operated eyes and the right eyes were the control eyes. The polysaccharide hydrogel 3 prepared in Example 9 was used as intraocular vitreous substitutes.

The experimental animals underwent routine animal anesthesia and skin disinfection, and vitrectomy surgery was performed to them. The first and second agents as prepared in Example 9 were injected into the vitreous cavity of the left eyes of the experimental rabbits through a dual syringe to form a gel in situ. The scleral incision was closed, and the experimental rabbits were cared for postoperatively, and were raised normally. The intraocular pressures of the rabbits were measured regularly after the operation. Slit lamp examinations of the eye, fundus photography, and B-ultrasound examination were conducted on the 90th day to evaluate the filling situation of the hydrogel in the vitreous cavity.

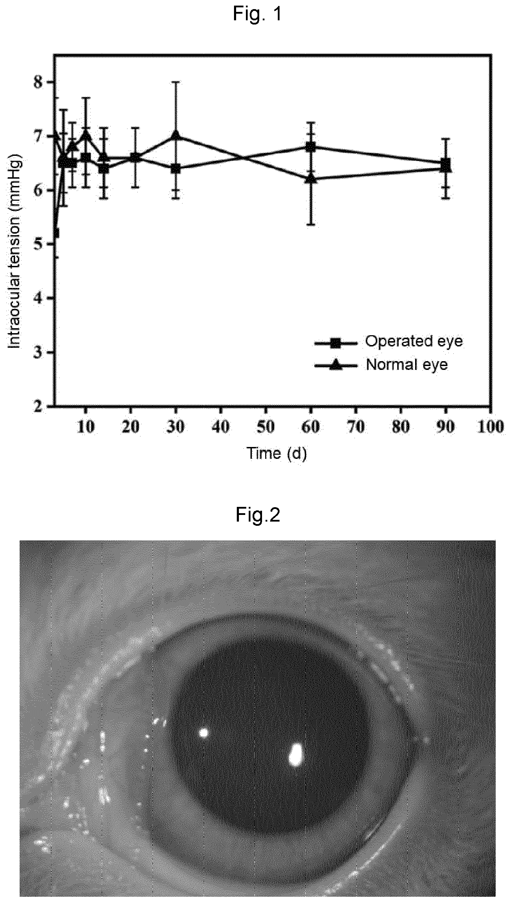

The results of the intraocular pressure measurement of the operated eye and the normal eye after the operation are shown in Figure 1. On the third day after the operation, the intraocular pressure of the operated eye was lower than that of the normal eye because the incision made on the sclera during the operation had not completely healed, causing a decrease in intraocular pressure. On the 7th day, the intraocular pressure of the operated eye basically returned to normal, and thereafter the intraocular pressure was basically stable, with no significant difference from the intraocular pressure of the normal eye, indicating that the polysaccharide hydrogel of the present invention played a good role in maintaining intraocular pressure. Compared with the patent application with publication number CN 105833344 A, the intraocular pressure of the operated eye according to the invention was more stable, indicating that the polysaccharide hydrogel of the present invention has a better effect on maintaining intraocular pressure.

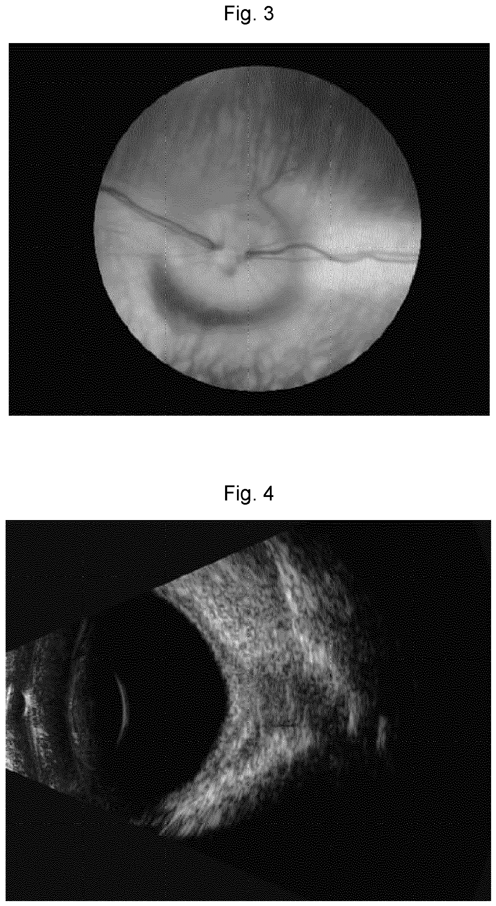

Observations by slit lamp on the 90th day after the operation are shown in Figure 2. The operated eye of the experimental rabbit showed no inflammatory reaction, the cornea was normal without edema, the vitreous remained transparent, and the fundus was clearly visible. Fundus photography image is shown in Figure 3. The blood vessels on the choroid were clearly visible, showing a reticular distribution, and there were no fundus lesions such as retinal and choroidal hemorrhage. B-ultrasound examination image is shown in Figure 4. No vitreous foreign bodies, cloudy bleeding, retinal detachment were detected, and the operated eye showed no significant difference compared with the normal eye. The experimental results show that the biological polysaccharide hydrogel prepared by the present invention has no irritant effect in the eye and has good ocular tissue compatibility. It has a very good vitreous substitution effect, can support the retina and maintain the shape of the eyeball, has good optical properties, and can degrade and be absorbed in the eye. The polysaccharide hydrogel has great potential for use as an intraocular vitreous substitute.

## Claims

1. A biological polysaccharide hydrogel formed by mixing and reacting a first solution with a second solution, wherein the first solution is a solution of a polysaccharide derivative with an aldehyde group on a side chain, and the second solution is a solution of a polysaccharide derivative with a primary amino group on a main chain or on a side chain, or a solution of a small molecule compound with two or more primary amino groups.

2. The biological polysaccharide hydrogel according to claim 1, wherein the first solution comprises 0.5% to 15% by weight of a polysaccharide derivative with an aldehyde group on a side chain, and the second solution comprises 0.5% to 15% by weight of a polysaccharide derivative with a primary amino group on a main chain or on a side chain, or 0.2% to 30% by weight of a small molecule compound with two or more primary amino groups; and the first and second solutions comprise a solvent selected from the group consisting of sterile water, physiological saline, physiological balanced solution, and glucose solution.

3. The biological polysaccharide hydrogel according to claim 1, wherein the polysaccharide derivative with an aldehyde group on a side chain in the first solution is an aminopolysaccharide derivative with an aldehyde group on a side chain or is a carboxypolysaccharide derivative with an aldehyde group on a side chain.

4. The biological polysaccharide hydrogel according to claim 3, wherein the aminopolysaccharide derivative with an aldehyde group on a side chain is an aminopolysaccharide derivative whose amino group is modified to form a side chain having an aldehyde group, and the carboxypolysaccharide derivative with an aldehyde group on a side chain is a carboxypolysaccharide derivative whose carboxyl group is modified to form a side chain having an aldehyde group.

5. The biological polysaccharide hydrogel according to claim 3, wherein the aminopolysaccharide derivative with an aldehyde group on a side chain is formed by oxidation of an aminopolysaccharide whose amino group is modified to form a side chain having an o-dihydroxyl group, and the carboxypolysaccharide derivative with an aldehyde group on a side chain is formed by oxidation of a carboxypolysaccharide whose carboxyl group is modified to form a side chain having an o-dihydroxyl group.

6. The biological polysaccharide hydrogel according to any one of claims 3 to 5, wherein the aminopolysaccharide derivative includes hydroxyethyl chitosan, hydroxypropyl chitosan, hydroxybutyl chitosan, carboxymethyl chitosan, or carboxyethyl chitosan; and the carboxypolysaccharide derivative includes a chitosan derivative with a carboxyl group, a chitin derivative with a carboxyl group, a hyaluronic acid with a carboxyl group or a hyaluronic acid derivative with a carboxyl group, chondroitin sulfate, heparin, or sodium alginate.

7. The biological polysaccharide hydrogel according to claim 6, wherein the chitosan derivative with a carboxyl group includes carboxymethyl chitosan, carboxyethyl chitosan, or succinyl chitosan; the chitin derivative with a carboxyl group includes carboxymethyl chitin or carboxyethyl chitin; the hyaluronic acid with a carboxyl group or the hyaluronic acid derivative with a carboxyl group includes hyaluronic acid, hydroxyethyl hyaluronic acid, hydroxypropyl hyaluronic acid, acetyl hyaluronic acid, butyryl hyaluronic acid, aminoacetic hyaluronic acid, aminopropionic hyaluronic acid or aminobutyric hyaluronic acid.

8. The biological polysaccharide hydrogel according to claim 1, wherein the polysaccharide derivative with a primary amino group on a main chain is an aminopolysaccharide derivative with both a primary amino group on the main chain and a side-chain grafted structure; the polysaccharide derivative with a primary amino group on a side chain is a polysaccharide derivative with both a carboxyl group on a main chain and a primary amino group on a side chain.

9. The biological polysaccharide hydrogel according to claim 8, wherein the polysaccharide derivative with a primary amino group on a side chain is formed by condensation reaction of a carboxyl group of a carboxypolysaccharide with one amino group of a small molecule compound with two or more primary amino groups.

10. The biological polysaccharide hydrogel according to claim 1, wherein the small molecule compound with two or more primary amino groups is an aliphatic or aromatic small molecule compound with two or more primary amino groups, including ethylenediamine, propylenediamine, butylenediamine, aminobutanamide, glutamine, alanyl-glutamine, lysine or other basic amino acids, a small molecule compound with a dihydrazide structure, diaminobenzoic acid or diaminophenylacetic acid.

11. The biological polysaccharide hydrogel according to claim 10, wherein the small molecule compound with a dihydrazide structure includes carbohydrazide, oxalyl dihydrazide, propanedioyl dihydrazide, butanedioyl dihydrazide, adipic dihydrazide or sebacic dihydrazide.

12. A method for preparing a biological polysaccharide hydrogel, which includes the following steps:
(1) providing a first solution, which is a sterilized solution comprising 0.5% to 15% by weight of a polysaccharide derivative with an aldehyde group on a side chain, and adjusting the pH of the first solution to 3.5 to 7, wherein the polysaccharide derivative with an aldehyde group on a side chain is formed by the reaction of an aqueous solution of a polysaccharide derivative having a grafted o-dihydroxyl group with an oxidizing agent;
(2) providing a second solution selected from the group consisting of:
i) a sterilized solution comprising 0.2% to 30% by weight of a small molecule compound with two or more primary amino groups;
ii) a sterilized solution comprising 0.5% to 15% by weight of a polysaccharide derivative with a primary amino group on a main chain;
iii) a sterilized solution comprising 0.5% to 15% by weight of a polysaccharide derivative with a primary amino group on a side chain;
and adjusting the pH of the second solution to 7 to 9, and measuring the molar percentage content of primary amino groups therein;
(3) mixing the first solution with the second solution: adding the first solution obtained in step (1) and the second solution obtained in step (2) respectively into tube A and tube B of a sterilized dual syringe, pushing them out from the syringe for mixing, and forming a transparent biological polysaccharide hydrogel by mixing the first solution and the second solution.

13. The method for preparing a biological polysaccharide hydrogel according to claim 12, wherein the polysaccharide derivative with an aldehyde group on the side chain in step (1) is prepared by the following method: providing an aqueous solution comprising 0.5% to 15% by weight of a polysaccharide derivative having a grafted o-dihydroxyl group, adding the oxidizing agent, wherein the molar ratio of the o-dihydroxyl group to the oxidizing agent is 1: (0.3 to 3), stirring the reaction mixture at room temperature or lower temperature and under dark condition for 2 minutes to 5 hours, adding an ethylene glycol solution to terminate the reaction for 0.5 to 2 hours, and performing dialysis of the reaction product and freeze drying, or precipitating with ethanol, washing, precipitating, and drying.

14. The method for preparing a biological polysaccharide hydrogel according to claim 12 or 13, wherein the polysaccharide derivative with a grafted o-dihydroxyl group in step (1) is prepared by the following method: providing an aqueous solution comprising 0.5% to 15% by weight of an aminopolysaccharide and an aqueous solution comprising 0.2% to 30% by weight of a small molecule compound with a carboxyl group and an o-dihydroxyl group respectively, adding the carboxyl activating agents 1-(3-dimethylaminopropyl)-3-ethylcarbodiimide hydrochloride and N-hydroxysuccinimide to the small molecule compound solution, and adjusting the pH to 3.5 to 7, stirring the resulting solution to react for 0.5 to 3 hours; adding the obtained small molecule compound solution to the aminopolysaccharide solution to form a reaction system, stirring and adjusting the pH of the reaction system to 7 to 9, and stirring the reaction system to react for 24 hours.

15. The method for preparing a biological polysaccharide hydrogel according to claim 14, wherein in step (1), the small molecule compound with a carboxyl group and an o-dihydroxyl group includes 2,3-dihydroxypropanoic acid, 2,3,4-trihydroxybutanoic acid, gluconic acid, threonic acid, arabic acid, xylonic acid, or mucic acid.

16. The method for preparing a biological polysaccharide hydrogel according to claim 12 or 13, wherein the polysaccharide derivative with a grafted o-dihydroxyl group in step (1) is prepared by the following methods: providing an aqueous solution comprising 0.5% to 15% by weight of carboxypolysaccharide and an aqueous solution comprising 0.2% to 30% by weight of a small molecule compound with an amino group and an o-dihydroxyl group respectively, adding the carboxyl activating agents EDC and NHS into the carboxypolysaccharide solution, adjusting pH to 3.5 to 7, stirring the resulting solution for 0.5 to 3 h; adding the small molecule compound solution into the carboxypolysaccharide solution to form a reaction system, stirring the reaction system and adjusting the pH thereof to 7 to 9, and stirring the reaction system to react for 24 h; performing dialysis of the reaction product and freeze drying, or precipitating the reaction product with ethanol, then washing, precipitating, and drying.

17. The method for preparing a biological polysaccharide hydrogel according to claim 16, wherein in step (1), the small molecule compound with an amino group and an o-dihydroxyl group includes aminopropylene glycol or aminoglucose.

18. The method for preparing a biological polysaccharide hydrogel according to claim 12, wherein the polysaccharide derivative with a primary amino group on a side chain in step (2) is prepared by the following steps: preparing an aqueous solution comprising 0.5% to 15% by weight of carboxypolysaccharide, and preparing an aqueous solution comprising 0.2% to 30% by weight of a small molecule compound with two or more primary amino groups, adding the carboxyl activating agents EDC and NHS into the carboxypolysaccharide solution, adjusting pH to 3.5 to 7, stirring the resulting solution for 0.5 to 3 h; adding the small molecule compound solution into the carboxypolysaccharide solution to form a reaction system, adjusting the pH to 7-9, and stirring the reaction system to react for 24 h; performing dialysis of the reaction product and freeze drying, or precipitating the reaction product with ethanol, then washing, precipitating, and drying.

19. The method for preparing a biological polysaccharide hydrogel according to any one of claims 14 to 18, wherein the molar ratio of the carboxyl groups to the carboxyl activating agents EDC and NHS is 1: (0.5 to 6): (0.5 to 6).

20. The method for preparing a biological polysaccharide hydrogel according to claim 12, wherein the oxidizing agent includes sodium periodate or potassium periodate.

21. Use of the biological polysaccharide hydrogel according to any one of claims 1 to 11 in the manufacture of an intraocular vitreous substitute.

22. An intraocular vitreous substitute comprising the biological polysaccharide hydrogel according to any one of claims 1 to 11.

23. The biological polysaccharide hydrogel according to any one of claims 1 to 11, which is used as an intraocular vitreous substitute.
